## Europäisches Patentamt
## European Patent Office
## Office européen des brevets

(11) Veröffentlichungsnummer: **0 266 474 B1**

(19)

# (12) EUROPÄISCHE PATENTSCHRIFT

(12)

(45) Veröffentlichungstag der Patentschrift: **31.03.93**

(51) Int. Cl.⁵: **A61F  11/00**

(21) Anmeldenummer: **86850390.5**

(22) Anmeldetag: **07.11.86**

(54) **Einrichtung zum Behandeln der Menièreschen Krankheit.**

(43) Veröffentlichungstag der Anmeldung:
**11.05.88 Patentblatt  88/19**

(45) Bekanntmachung des Hinweises auf die
Patenterteilung:
**31.03.93 Patentblatt  93/13**

(84) Benannte Vertragsstaaten:
**AT BE CH DE ES FR GB GR IT LI LU NL SE**

(56) Entgegenhaltungen:
**WO-A-83/02556**
**AT-B- 175 663**
**DE-C- 809 579**
**FR-A- 393 451**

(73) Patentinhaber: **DENSERT, Barbara**
**Broddesonsgatan 22**
**S-302 34 Halmstad(SE)**

Patentinhaber: **DENSERT, Ove**
**Broddesonsgatan 22**
**S-302 34 Halmstad(SE)**

(72) Erfinder: **DENSERT, Barbara**
**Broddesonsgatan 22**
**S-302 34 Halmstad(SE)**
Erfinder: **DENSERT, Ove**
**Broddesonsgatan 22**
**S-302 34 Halmstad(SE)**

(74) Vertreter: **Linderoth, Margareta et al**
**AB Astra Patent Department**
**S-151 85 Södertälje (SE)**

**Beschreibung**

Die Erfindung bezieht sich auf einen Apparat zur Erzeugung und Transmission von vorbestimmbaren komplexen Druckstössen zum Flüssigkeitssystem des Innenohres zwecks Erzielung einer intermittenten Kompression des Flüssigkeitssystemes im Hautlabyrinth des Innenohres.

Deutlicher ausgedrückt bezieht sich die Erfindung auf einen Apparat zur Herstellung von komplexen, reproduzierbaren Druckstössen zur Beeinflussung des im Innenohr vorhandenen Flüssigkeitssystemes über ein an den Gehörgang des Ohres von außen lösbar, anschliessbares Verbindungsstück und eine mit Steuermitteln zusammenwirkende, druckerzeugende Vorrichtung zur Erzeugung der reproduzierbaren Druckstösse mit Steuermitteln zur Steuerung der druckerzeugenden Vorrichtung mittels Regelorganen.

Der erfindungsgemäss vorgeschlagene Apparat kann mit Vorteil zur Beeinflussung des relativen Verhältnisses zwischen den beiden Flüssigkeiten (perilymphatische und endolymphatische Spatie) des Innenohres bei einem Zustand verwendet werden, bei welchem eine Schwellung des Hautlabyrinthes vorliegt (Meniere's Krankheit).

Die Erfindung kann auch für Studien der Effekte von verschiedenen Sorten von Druckwellen in beispielsweise Infraschall auf die Funktion des Innenohres bei Tieren und Menschen verwendet werden.

Es ist schon bekannt, dass Veränderungen in der endolymphatischen Spatie die Funktionseigenschaften des Ohres beeinflussen und gewisse Krankheiten sind auch hierauf zurückgeleitet worden. Ein Biespiel ist die sogenannte Meniere's Krankheit mit solchen Symptomen wie kraftige Gehörverminderung, Schwindel, Ohrensausen, Gefühle wie Blockierung des Ohres usw. Derartige Veränderungen ergeben in manchen Fällen ein vollständiges Arbeitsunvermögen und dies nicht nur auf Grund der herabgesetzten Funktionen sondern in hohem Ausmass auf Grund der Beeinflussung der Psyche, was in gewissen Fällen von sehr drastischem Charakter sein kann. Man hat früher schon versucht, die endolymphatische Spatie durch chirurgische Eingriffe zu beeinflussen, solche Eingriffe haben jedoch einen kurzzeitigen Effekt oder überhaupt keinen Effekt erwiesen.

Nach dem britischen Patent 10 695 von 1908 wurde schon ein vibrierender Apparat zur Behandlung von Krankheiten im Trommelfell vorgeschlagen um beispielsweise Kopfschmerzen ("head noices") und andere undefinierte Formen der Gehörherabsetzung zu beeinflussen. Gemäss der Beschreibung dieses britischen Patentes werden hierbei sowohl positive wie auch negative Drücke verwendet, eine Tatsache die wie aus nachstehender Beschreibung hervorgeht jedoch nicht zur Lösung des vorliegenden Problemes herangezogen werden kann.

Neuzeitlich wurde auch die Beeinflussung des hydrodynamischen Systemes des Innenohres durch in den Gehörgang applizierbare Druckveränderungen diskutiert. Die hierbei verwendete Vorrichtung ist in WO-A-83/02556 beschrieben und besteht aus ersten Organen zur Erzeugung eines statischen Druckniveaus, zweiten Organen zur Variation des statischen Druckes sowie dritten Organen zur Zusammenkupplung der ersten und zweiten Organe mit dem Ohrenkanal. Auch wenn man nach diesem neuartigen Vorschlag versucht hat, das hydrodynamische System im Innenohr zu beeinflussen, ergibt dieser Vorschlag keine praktisch verwendbare Lösung des vorliegenden Problemes weil der notwendige und erfindungsgemäss vorgeschlagene Aufbau von besonderen, komplexen Druckstössen aus dieser Publikation nicht ausgelesen werden kann.

Es wurde nun überraschend festgestellt, dass man erfindungsgemäss die Nachteile bisher bekannter Vorschläge ausräumen kann und hauptsächlich kennzeichnend für den erfindungsgemäss vorgeschlagene Apparat ist, daß eine erste Druckkomponente (a) mit variierenden Druckschwakungen kurzer Pulslänge und eine zweite, der erstgenannten Komponente (a) überlagerten pulsartige Komponente (b) mit einem vorbestimmten Überdruck und mit einer größeren Pulslänge als der der ersten Druckkomponente (a) erzeugt wird, wobei die Summe der genannten ersten und zweiten Komponenten immer positiv ist.

Übrige Eigenschaften der Erfindung gehen aus den in den beigefügten Ansprüchen angegebenen Kennzeichen hervor.

Grundliegend für die Erfindung ist die Beobachtung, dass die periphere Gehör- und Balanzfunktionen zum Innenohr lokalisiert sind, dass aus einer Beinkapsel besteht, die Membrane und Sinnenzellen enthält sowie mit zwei verschiedenen Flüssigkeiten gefüllt ist (peri- und endolymphatische Spatie). Die Flüssigkeiten des Innenohres transportieren Lautenergie innerhalb des Innenohres und fügen in dieser Weise die verschiedenen Strukturen des Innenohres funktionell zusammen. Der relative Volumen der Flüssigkeiten und deren chemische Zusammensetzung ist von ausschlaggebender Bedeutung für die Funktion des Innenohres. Eine Zunahme der Flüssigkeit des Hautlabyrinthes (endolymphatische Spatie) ergibt eine Anzahl von Innenohrssymptomen, wie herabgesetzte Gehörfunktionen, Schwindel und ein Druckgefühl im Ohr (Meniere's Krankheit). Der Ausmass dieser Beschwerden kann direkt auf eine erhöhte Menge dieser Flüssigkeit relatiert werden.

EP 0 266 474 B1

Das hydrodynamische System des Innenohres ist mit einer Anzahl von druckregelnden Mechanismen ausgerüstet, die eine normale Funktion unter physiologischen Druckvariationen des Innenohres zulassen. Das druckausgleichende Vermögen des Innenohres bezieht sich darauf, dass die Flüssigkeiten des Innenohres sowie die intravaskulären Flüssigkeiten über sogenannte Druckausgleichkanäle bei beispielsweise Veränderungen von Körperlagen, Blutdruck sowie bei Husten umverteilt werden können. Durch Tierversuche konnte eine gewisse Erkenntnis über die Kapazität und die Voraussetzung für Flüssigkeitsströmungen durch diese Kanäle erreicht werden. Es hat sich erwiesen, dass man durch die erfindungsgemäss vorgeschlagene komplexe Druckpulse eine erhöhte Flüssigkeitsströmung durch gewisse von diesen genannten Kanälen erzwingen kann.

Diese differenzierte Flüssigkeitsströmung sollte durch vorliegende Unterschiede zwischen den verschiedenen Kanälen möglich sein, d.h. deren druckregelnde Kapazität sowie durch die Tatsache, dass die totale Flüssigkeitsmenge innerhalb der steifen Wände der Kapsel des Innenohres die gleiche bleiben muss. Da die beiden Flüssigkeitssysteme (peri- und endolymphatische Spatie) anatomisch gänzlich von einander unterschieden sind und auch mit eigenen Druckausgleichkanälen versehen sind, d.h. Wege zur Flüssigkeitsströmung, sollte es erfindungsgemäss möglich sein, mit Hilfe von komplexen Druckpulsen die Menge der endolymphatischen Flüssigkeit zu reduzieren ohne dass hierbei gleichzeitig die perilymphatische Flüssigkeitsmenge nennenswert beeinflusst wird. Erfindungsgemäss erreichte Effekte zeigen hierauf hin bei Meniere's Krankheit, da die erhöhte Endolymphamenge direkt proportional zur Funktion des Innenohres ist. Zwecks Erzielung des erfindungsgemäss angestrebtene Effektes, d.h. in so grossem Ausmass wie möglich einen Abfluss durch die endolymphatischen Kanäle zu erreichen, muss der hauptsächliche Kanal für perilymphatische Strömung, das sogenannte COCHLEAR AQUEDUCT ausser Spiel gesetz werden.

Klinische Versuche mit der erfindungsgemäss vorgeschlagenen Technik haben erwiesen, dass die Flüssigkeitsströmung individuell variiert auf Grund der anatomischen Ausformung und der funktionellen Kapazität, weshalb die verschiedenen Parametern der Druckstösse variiert werden müssen, so dass die Strömung durch das Aquädukt gehemmt wird gleichzeitig wie eine generelle Druckzunahme die Strömung durch andere Ausströmungswege stimuliert. Somit hat es sich erfindungsgemäss gezeigt, dass eine dem Grunddruck überlagerte Sinuswelle eine turbulente Strömung im Abfluss erzielt, wodurch eine partielle funktionelle Blockade des Abflusses erreicht wird. Diese Blockade ist auf Grund der oben beschriebenen individuellen Unterschiede in der Anatomie des Abflusses auf einen beschränkten Zeitverlauf gebunden (d.h. die Strömung durch den Abfluss kann mit Zeitkonstanten beschrieben werden). Die Länge der Druckstösse und der Intervall zwischen zwei aufeinander folgenden Druckstössen ist deshalb von grosser Bedeutung und sollte individuell angepasst werden können. Die Sinuswellenfrequenz soll variiert werden können da es auf Grund von Resonanzphänomenen erwartet wird, dass gewisse Frequenzen effektivere als andere sind. Erfindungsgemäss ist es somit möglich, die Variationen der totalen Druckamplitude zum Niveau anzupassen, wo meist möglichen Umverteilung der Flüssigkeit zwischen den intra- und extralabyrinthären Räumen stattfindet.

Während die Applikation eines positiven Druckpulses den Abfluss der Endolympha stimulieren kann und die endolymphatische Schwellung reduzieren kann, kann auch das umgekehrte Verhältnis, d.h. Zufuhr eines negativen Druckpulses, eine erhöhte Zufuhr von endolymphatischer Flüssigkeit mit nachfolgender Verschlechterung und erhöhten Beschwerden mitführen. Deshalb sollte ein System zur Transmission eines komplexen Druckstosses erfindungsgemäss zweckmässig mit einer Sicherheitsvorrichtung versehen sein, die einen negativen Druckstoss unmöglich macht.

Da praktisch kein direktes Verfahren zum Messen des Flüssigkeitsdruckes im Innenohr eines Menschen existiert, sollte ein indirektes Mass der hydrodynamischen Balance des Innenohres bei Menschen verwendet werden falls das Innenohr für die erfindungsgemäss vorgeschlagenen Druckstösse ausgesetzt wird. Eine klinische-audiologische Auswertungsmethode wurde hierzu vorgeschlagen und besteht aus Observationen der subjektiven Symptome des Patienten wie Schwindel und Druckgefühl im Ohr sowie von Messungen der Gehörfunktion. Die Gehörfunktion wird mit Hilfe der klassichen audiologischen Prüfbatterie ausgewertet, sowie mit besonders für diesen Zweck konstruierten Methoden zur Messung von audiologischer Signalanalyse. Die letztgenannte Methode kann also auch zur Messung von Psychoakustischen Tuning Curves (PTC) ausgenützt werden und kann das Ausmass der Störung die dem signalanalytischen Teil des Innenohres trifft quantitativ angeben. Die Störung in dieser Funktion kann zum Ausmass der Schwellung innerhalb des Hautlabyrinthes bei Meniere's Krankheit relatiert werden.

Das oben beschriebene Modell zur Auswertung der Funktionen des Innenohres im Zusammenhang mit Exponierung von Druckstössen ist äusserst notwendig und ein wichtiger Teil bei der Wahl von adäquaten Druckparametern und muss deshalb als wichtiger Teil in die Behandlung eingenommen werden.

Die Erfindung wird nachstehend an Hand beigefügter Zeichnungen näher beschrieben, in welche

Figur 1  ein mathematisches Modell des erfindungsgemäss vorgeschlagenen Prinzips, und

3

Figure 2    eine schematische Ansicht eines erfindungsgemäss vorgeschlagenen Apparates darstellt.

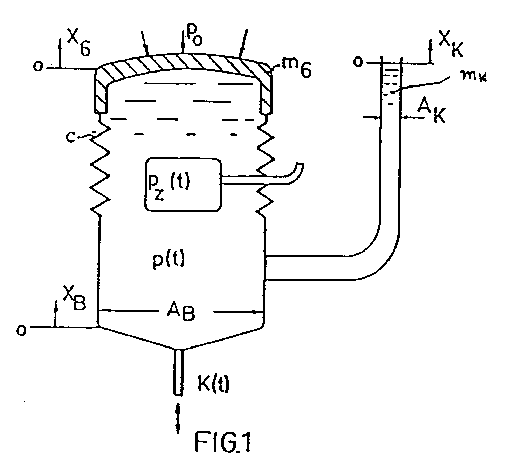

Die Wirkungsweise der Erfindung bei dem aktuellen Verwendungsgebiet ist noch nicht gänzlich geklärt. Höchst wahrscheinlich gelten jedoch die Grundsätze die nachstehend an Hand des in der Fig. 1 gezeigten mathematischen Modelles erklärt sind.

Grundlegend befindet sich im Inneren eines Ohres ein Flüssigkeitslabyrinth bestehend aus einem inneren System, das aus einer Haut einer semipermeabeln Membrane besteht, die mit Gewebeflüssigkeit gefüllt ist. Dieses innere System schwebt oder kann so zu sagen gänzlich von einem äusseren System umgegeben sein und ist mit der Aussenwelt durch Membrane oder Kanäle usw. verbunden. Das äussere System ist im Körper elastisch gelagert und gleich mit Gewebeflüssigkeit gefüllt und zeigt einen Kanalabfluss nach Aussen auf.

Durch die Erfindung wird grundsätzlich beispielsweise durch einen zweckmässigen, hierzu angepassten Druckwellengenerator am Aussensystem eine periodische Kraft K (t) erzeugt, die im Rahmen der Elastizität in Form eines Pumpschlages den Druck in der Flüssigkeit im Aussensystem erhöht. Hierdurch wird das Aussensystem mit seiner Gewebemasse $m_6$ verschoben, so dass die verdrängte Gewebemasse entsprechend der Kontinuität ausreichend Raum findet. In der Regel wird dieser Raum teilweise dadurch geschaffen, dass die Gewebeflüssigkeit über den Kanalquerschnitt $A_X$ abströmt. Die Aussenhülle wird hier bezüglich der Elastizität durch die Konstante C gekennzeichnet. Falls man sich nun vorstellt, dass die untere Fläche $A_B$ periodisch auf und ab bewegt wird, so wird bei niedrigen Frequenzen deres periodischen Verlaufes die Aussenhülle entsprechend $X_6$ und die Flüssigkeitssäule entsprechend $X_K$ (Kanal) verschoben. Die am Boden mit der Fläche $A_B$ und dem Weg $X_B$ verdrängte Flüssigkeit finden wir am Kopf bei $A_B$ und $X_6$ bzw. bei $A_K$ und $X_K$ wieder.

Ohne zunächst auf das Innensystem zu achten kann die harmonische Bewegung der folgenden drei Systeme beobachtet werden.

1) An der speziellen Aussenstelle der Hülle wird die Erregerkraft K (t) (t = Zeit) auf und ab gehen.

2) Synchron dazu hebt bzw. dehnt sich das Aussensystem mit der Masse $m_6$.

3) Synchron dazu schwingt die Kanalgewebesäule mit der Masse $m_K$ hin und her.

Mittels der erfindungsgemäss vorgeschlagenen Beeinflussung des Aussensystemes soll eine Druckfunktion im Aussensystem derart konzipiert werden, dass dessen Abströmung über die Aussenkanäle verhindert wird und gleichzeitig ein kräftiger Druckstoss auf das Innensystem so appliziert werden kann, dass Strömungswiederstände in den Abflusswegen des Aussensystemes überwunden werden können und ein geringer Abfluss von Gewebeflüssigkeit bewirkt wird.

1. Aussensystem

Ermittels des vorgeschlagenen Systemes können die massgebenden Differentialgleichungen für die Bewegung der Gewebemasse $m_6$ bzw. für die Bewegung der Kanalsäule mit der Masse $m_K$ aufgestellt werden. Setzt man für die erzeugte Schwingung durch den erfindungsgemäss vorgeschlagenen Schwunggenerator die folgende harmonische Funktion

$$X_B = A^* \sin \omega t + g(t) \qquad (1)$$

wobei $\omega$ die Erregerfrequenz bezeichnet, wird folgende Differentialgleichung für die Gewebemasse erhalten

$$\ddot{X}_6 + \frac{c}{m_6} \cdot X_6 = \frac{A_B}{m_6} \left[ p(t) - p_o \right] \qquad (2)$$

wobei die Dämpfung vernachlässigt wurde und zudem vereinfachte Randbedingungen gewählt wurden. Entsprechend erhält man für die Kanalmasse $m_K$

$$\ddot{X}_K = \frac{A_K}{m_K} \left[ p(t) - p_o \right] \qquad (3)$$

die Kontinuitätsgleichung ergibt weiter

$$A_B \cdot X_B = A_B \cdot X_6 + A_K \cdot X_K \qquad (4)$$

woraus nach einigem Umformen und nach bestimmten Transformationen die folgende Differentialgleichung für die Kanalmasse erreicht wird

$$\ddot{X}_K + \beta X_K = \alpha \ddot{X}_B + \delta X_B \qquad (5)$$

Hier bedeuten

$$\beta = f \cdot \alpha \cdot \frac{c}{m_6} \qquad \text{und} \quad \delta = \alpha \cdot \frac{c}{m_6} \qquad (6) \quad \text{und} \qquad (7)$$

mit

$$\alpha = \frac{f}{f^2 + \mu} \quad , \quad f = \frac{A_K}{A_B} \quad \text{und} \quad \mu = \frac{m_K}{m_6} \qquad (8), \ (9), \ (10)$$

c steht für die Federkennzahl der elastischen Aussenhülle. In Gleichung (1) stellt A* die Amplitude der harmonischen Schwingverformung der Aussenmembrane dar und g (t) stellt eine Zusatzfunktion dar, die später mit einem zeitlich begrenzten Druckstoss identifisiert werden kann. Vernachlässigt man zunächst die Stossfunktion g (t), so läss sich die homogene Differentialgleichung zu Gleichung (5) lösen und man erhält

$$X_{K,H} = a \cdot \sin [\sqrt{\beta} \cdot t] \qquad (11)$$

wobei mit den gegebenen Randbedingungen für die Amplitude

$$a = A^* \frac{w/w_E}{1 - (w/w_E)^2} \left[ \frac{1}{f} - \alpha \cdot \left( \frac{w}{w_E} \right)^2 \right] \qquad (12)$$

hergeleitet werden kann. Die Eigenfrequenz des schwingungsfähigen Systemes ist identisch mit $\sqrt{\beta}$ und deshalb gilt

$$\omega_E = \sqrt{\beta} \qquad (13)$$

Das Verhältnis von Erregerfrequenz $\omega$ zur Erregerfrequenz $\omega_E$ hat hier wie üblich eine grosse Bedeutung. Eine partikuläre Lösung der Differentialgleichung (5) kann über

$$X_{K,I} = B \cdot A^* [-\alpha\omega^2 + \delta]\sin \omega t \qquad (14)$$

mit

$$B = \frac{1}{\beta - \omega^2} \qquad (15)$$

gefunden werden, so dass die Gesamtlösung lautet

$$X_K = a \cdot \sin \omega_E t + \frac{1}{\omega_E^2 - \omega^2} \cdot A* \left[ -\alpha \omega^2 + \delta \right] \sin \omega t \qquad (16)$$

über die Geschwindigkeit der Kanalmasse $\dot{X}_K$ erhält man für die zeitliche Massenströmung

$$\dot{m}_K = \rho \cdot A_K \cdot \dot{X}_K \qquad (17)$$

mit der Gewebeflüssigkeitsdichte $\rho$. Nach Substitution folgt

$$\frac{\dot{m}_K}{\rho \cdot A_K \cdot A* \cdot \omega_E} = \frac{\omega / \omega_E}{1 - (\omega / \omega_E)^2} \left[ \frac{1}{f} \alpha - \left(\frac{\omega}{\omega_E}\right)^2 \right] \left\{ \cos \omega_E t + \cos \omega t \right\} \qquad (18)$$

so dass man hierdurch eine Aussage über die Massenströmung im Auslaufkanal machen kann. Diese Abströmung nähert sich Null wenn

$$\frac{1}{f} = \alpha \left(\frac{\omega}{\omega_E}\right)^2 \qquad (19)$$

Der Schwinggenerator muss somit bei einer Erregerfrequenz $\omega$ wie folgt eingestellt werden

$$\frac{\omega}{\omega_E} = \sqrt{1 + \left(\frac{A_B}{A_K}\right)^2 \cdot \frac{m_K}{m_6}} \qquad (20)$$

Da die Gewebeaussenfläche $A_B$ im Verhältnis zur Kanalfläche $A_K$ sehr gross ist, wird der quadratische Einfluss dieses Verhältnisses dominant.

Die Kanalmasse $m_K$ ist allerdings viel geringer als die Gewebemasse im Aussensystem. Alles in allem ist das Frequenzverhältnis $\omega/\omega_E > 1$, was logischer Weise und wie erwartet überkritisch liegt. Damit muss der Schwinggenerator bei einer Erregerfrequenz $\omega$ eingestellt werden, die über der Eigenfrequenz

$$\omega_E = \sqrt{f \cdot \alpha} \cdot \sqrt{\frac{c}{m_6}} \qquad (21)$$

liegt. Es ist anzunehmen, dass diese Eigenfrequenz sehr niedrig liegt, weil die Federsteifigkeit c vermutlich klein ist, die Gewebeflüssigkeit $m_6$ im Aussensystem relativ gross und das Flächenverhältnis f auch sehr klein ist.

Die Lösungsgleichung (18) für die Kanalmassenströmung beinhaltet noch nicht den momentanen, zeitlich begrenzten Stosseinfluss, weil die mathematische Behandlung sehr komplex ist.

## 2. Innensystem

Eine geschlossene Lösung der Differentialgleichung mit der Stossfunktion g (t) ist sehr kompliziert und ist deshalb nicht näher spezifiziert worden. Die aus der Gleichung (3) nun überblickbare Druckfunktion

$$p\ (t)\ -\ p_O\ =\ \frac{\ddot{x}_K\ \cdot\ m_K}{A_K} \qquad\qquad (22)$$

wird gemäss der am Schwinggenerator eingestellten Amplitude A* die absoluten Drücke in der Aussengewebeflüssigkeit steuern. Durch den überlagerten Druckstoss könnte dann kurzzeitig ein Spitzendruck erzeugt werden, der durch seine Einwirkung auf das zentrale System die gewünschte Flüssigkeitsausströmung erzielen könnte. Natürlich müsste der gewählte Druckstoss optimiert werden. Wenn die Kanalmasse nach hinten strömt, wird somit der Druckstoss gegen die Innenkammer appliziert, wobei eine Komprimierung dieser erzielt wird. Im Aussensystem würde auf Grund der rückströmenden Kanalmasse nur eine erhöhte elastische Dehnung der Gewebemembrane erreicht werden. Der Abfluss bleibt gesperrt und auf Grund des Stosses wird eine schnelle Dämpfung des stabilen harmonischen Schwenkungszustandes erreicht.

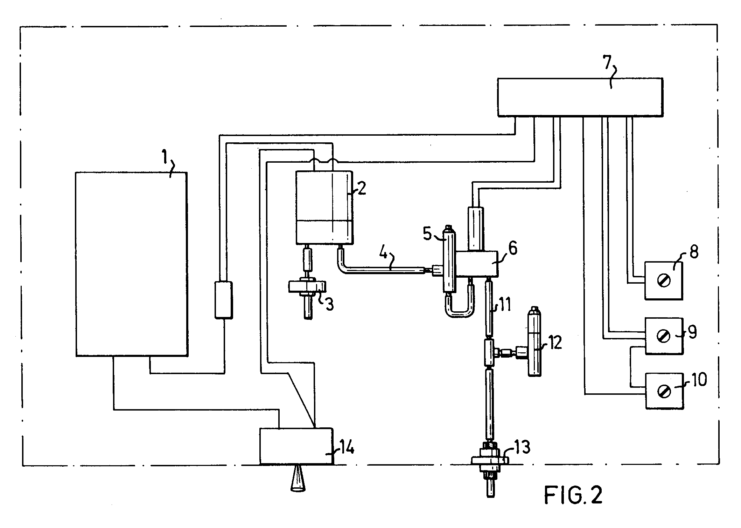

In Fig. 2 wird eine prinzipielle Skizze eines erfindungsgemäss ausgebildeten Apparates gezeigt, wobei 1 eine Stromquelle bezeichnet - zweckmässig bestehend aus einer Anzahl von Batterien. Mit 2 wird eine druckerzeugende Vorrichtung bezeichnet, beispielsweise in Form einer Membranpumpe oder Rotationspumpe. Bei Verwendung einer Rotationspumpe kann zweckmässig ein Schalldämpfer 3 verwendet werden. Der in der Pumpe erzeugte Druck wird über die Leitung 4 und einen als Drossel ausgebildeten Einwegventil zu einem Dreiwegeventil 6 geleitet, welches bezüglich der Frequenz und Länge der gewünschten Druckstösse von einer Anzahl Elektronkreisen 7 über Potentiometern 8, 9 und 10 gesteuert wird.

Der so erhaltene komplexe Druckstoss wird über die Leitung 11 über ein einstellbares, als Leckventil dienendes Ventil 12 über zweckmässige Anschlüsse 13 zum Innenkanal des Ohres geleitet.

Jeder Apparat wird zuerst bezüglich der Länge, Frequenz und Dauer des Druckstosses durch Versuche innerhalb eines allgemein verwendbaren Gebietes eingestellt und danach für jeden Patient besonders eingestellt bevor der Apparat zum Verbraucher für kontinuierlichen Gebrauch abgegeben wird.

Der Apparat besteht somit aus einem elektronischen und einem pneumatischen Teil. Die Elektronik umfasst zwei Kreiskarten. Die erste Kreiskarte steuert die Länge des Pulses und das Intervall zwischen den Pulsen. Zweckmässig kann hierbei die Pulslänge und die Pauslänge jeweils für sich von etwa 0 bis 20 Sekunden variieren.

Die andere Kreiskarte steuert die Druckmodulation und bestimmt auch die Frequenz der Modulation, die zweckmässig zwischen 1 bis 20 Hz variiert werden kann. Diese beiden Kreiskarten steuern auch einen elektrisch-pneumatisches Zweiwegeventil mit welchem der Luftstrom zum oder vom Ohr variiert werden kann. Das pneumatische Teil besteht somit aus dieser Pumpe, die den Luftstrom erzeugt, und dem obengenannten Ventil.

Ausserdem kann der Luftstrom durch das oben beschriebene Einwegventil reguliert werden oder durch einen Geschwindigkeitsregulator der die Drehzahl der Pumpe steuert oder eine Kombination hiervon.

Das obengenannte Einwegventil regelt die totale Amplitude des Druckes.

Der Apparat kann mit Batterien ausgerüstet werden oder auch kann für die Stromversorgung ein gewöhnlicher Anschluss verwendet werden. Mit 14 wird ein Stromschalter bezeichnet.

Die aktuelle Konstruktion lässt eine Verwendung eines Frequenzspektrums von 3 bis 20 Hz zu.

Um zu untersuchen ob die Effekte der aktuellen Druckpulse der bisher bekannten Technik überlegen waren wurden etwa 10 Patienten mit den erfindungsgemässen Druckpulsen behandelt.

Die totale Amplitude des Druckpulses variierte von 10 bis 30 Cm Wassersäule und die Amplitude der Modulierung von 25 bis 0 Cm Wassersäule. Die Frequenzen variierten von 3 bis 15 Hz und die Länge des Pulses konnte von 0,5 bis 5 Sekunden variieren. Die Pulse wurden jede 4,5 Sekunde initiiert und das Ohr wurde in dieser Weise zwischen 5 bis 10 Minuten behandelt, wonach diese Behandlung anfangs jede zweite Stunde wiederholt wurde. Nach Erreichen des klinischen Effektes wurde die Frequenz der Behandlung auf 3,4 Hz pro Tag vermindert.

Die Druckparametern wurden stufenlos variiert. Die Zeiten und Frequenzen der Behandlung variierten innerhalb der oben angegebenen Grenzen, doch individuell. Bei den Versuchen wurden Patienten ausgewählt, mit sehr variierenden Hörverlusten, mit Individen mit sehr gravierenden Hörverminderungen.

Es wurde festgestellt, dass die klinische Verbesserung in der Regel schon nach einigen Tagen oder Stunden nach beginnender Behandlung eintritt. Diese Verbessungen beziehen sich auf Schwindel, Druckgefühl und Hörfunktion.

Hierbei vorgenommene objektive Messungen der Hörfunktion zeigten signifikante Verbesserungen.

Nach präliminaren Erfahrungen von diesen Versuchen wurde beobachtet, dass die Ausrüstung leicht zu hantieren ist und noch aus therapeutischem Gesichtpunkt sehr effektiv ist, was etwa wie folgt erklärt werden könnte:

Der eingestellte Druck und damit der technische Effekt konnte während der Behandlung konstant gehalten werden. Man konnte somit instabile Drücke verhindern.

Der Zugang zur Verwendung von verschiedenen Frequenzen des Pulses und der Modulation ergibt grössere Möglichkeiten, die Resonanzfrequenz zu finden, die für jeden Patient optimal angesehen werden kann und die individuell variiert in Abhängigkeit von der Grösse und der Kapazität der Druckausgleichkanäle des Innenohres.

Durch die erfindungsgemäss vorgeschlagene Konstruktion ergeben sich gute Möglichkeiten zur Variation der Parametern des Druckpulses. Die für den Individen spezifike und optimale Kombination von Parametern kann hergestellt werden, geprüft und auch sicher dem Patienten zugeschleusst werden.

Der Unterschied zwischen einer erhaltenen klinischen Verbesserung und einer nicht erreichten Verbesserung und auch Verschlechterung liegt darin, dass man für jeden Patienten eine optimale Kombination von sämtlichen Parametern des Druckes finden kann, die eine Strömung von Endolympha durch den Endolymphakanal vom Hautlabyrinth ergibt, und wobei gleichzeitig die Strömung durch cochleäre Aquädukte funktionell blockiert wird. Der Punkt für die funktionelle Blockade erwies sich durch eine Serie von Tierexperimenten als sehr kritisch, d.h. abhängig von sehr kleinen Variationen in den Parametern des Druckes.

Die Erfindung ist natürlich nicht auf den in Fig. 2 gezeigten Apparat beschränkt, sondern kann in verschiedene Weise im Rahmen der nachfolgenden Patentansprüche variiert werden.

**Patentansprüche**

1.  Apparat zur Herstellung von komplexen, reproduzierbaren Druckstössen zur Beeinflussung des im Innenohr vorhandenen Flüssigkeitssystemes über ein an den Gehörgang des Ohres von außen lösbar, anschliessbares Verbindungsstück und eine mit Steuermitteln (7) zusammenwirkende, druckerzeugende Vorrichtung (2) zur Erzeugung der reproduzierbaren Druckstösse mit Steuermitteln (7) zur Steuerung der druckerzeugenden Vorrichtung (2) mittels Regelorganen (6), dadurch **gekennzeichnet** daß
    eine erste Druckkomponente (a) mit variierenden Druckschwakungen kurzer Pulslänge und
    eine zweite, der erstgenannten Komponente (a) überlagerte pulsartige Komponente (b) mit einem vorbestimmten Überdruck und mit einer größeren Pulslänge als der der ersten Druckkomponente (a) erzeugt wird,
    wobei die Summe der genannten ersten und zweiten Komponenten immer positiv ist.

2.  Apparat nach Anspruch 1, **gekennzeichnet** durch so eingerichtete Regelorgane, daß die zweite Komponente (b) nach Erreichen eines Peakeffektes der ersten Druckkomponente (a) abgegeben wird.

3.  Apparat nach Anspruch 1 oder 2, dadurch **gekennzeichnet**, daß Organe zur Abgabe von Pulslängenvariationen von zwischen 0,5 und 5 Sek. und zum Initiieren von Pulsen zwischen 1-6 Sek. vorgesehen sind.

**Claims**

1.  Apparatus for producing complex, reproducible pressure pulses to influence the fluid system present in the inner ear by means of a connecting part which is releasably attachable to the auditory canal of the ear from outside and a pressure-generating device (2), cooperating with control means (7), to generate the reproducible pressure pulses with control means (7) to control the pressure-generating device (2) by means of regulating elements (6), characterised in that
    a first pressure component (a) with varying pressure fluctuations of short pulse length and
    a second, pulsed component (b), superposed above the first mentioned component (a), with a predetermined excess pressure and with a greater pulse length than that of the first pressure component (a) are generated,
    the sum of the said first and second components always being positive.

2. Apparatus according to Claim 1, characterised by regulating elements which are arranged so that the second component (b) is given off after reaching a peak effect of the first pressure component (a).

3. Apparatus according to Claim 1 or 2, characterised in that elements are provided to give off pulse length variations of between 0.5 and 5 sec and to initiate pulses between 1-6 sec.

**Revendications**

1. Appareil pour produire des coups de bélier complexes et reproductibles, destinés à influer sur le système de fluides se trouvant dans l'oreille interne, par l'intermédiaire d'une pièce de jonction pouvant être raccordée au conduit auditif de l'oreille et pouvant en être détachée de l'extérieur, et d'un dispositif générateur de pression (2), coopérant avec des organes de commande (7), destiné à produire les coups de bélier reproductibles, et comportant des organes de commande (7) destinés à commander le dispositif générateur de pression (2) à l'aide d'organes de régulation (6), caractérisé en ce qu'on produit
   une première composante de pression (a) présentant des fluctuations variables de pression de faible longueur d'impulsions, et
   une deuxième composante (b), pulsée, superposée à la composante (a) mentionnée ci-dessus, présentant une surpression prédéfinie et ayant une longueur d'impulsion supérieure à celle de la première composante de pression (a),
   la somme de la première et de la deuxième composantes ci-dessus étant toujours positive.

2. Appareil selon la revendication 1, caractérisé par des organes de régulation arrangés de façon que la deuxième composante (b) soit émise après qu'a été atteint un effet de maximum (effet de pic) de la première composante de pression (a).

3. Appareil selon la revendication 1 ou 2, caractérisé en ce qu'on prévoit des organes destinés à émettre des variations de longueur d'impulsions de 0,5 à 5 secondes et à déclencher des impulsions de 1 à 6 secondes.

FIG.1

FIG.2